# EUROPEAN PATENT APPLICATION

(11) **EP 4 620 979 A1**
(43) Date of publication of application: **24.09.2025**
(21) Application number: 23891541.7
(22) Date of filing: 13.11.2023
(51) Int. Cl.: C07K 16/32, A61K 39/395, A61P 35/00, C12N 15/13

(54) **ANTIBODY AND METHOD FOR PRODUCING ANTIBODY**

(30) Priority: 14.11.2022 JP 2022182045
(71) Applicant: Cognano, Inc., Kyoto-shi, Kyoto 601-1255 (JP)
(72) Inventor: MAEDA, Ryota, Kyoto-shi, Kyoto 601-1255 (JP); YAMAZAKI, Hiroyuki, Kyoto-shi, Kyoto 601-1255 (JP); IMURA, Akihiro, Kyoto-shi, Kyoto 601-1255 (JP)
(74) Representative: Mathys & Squire
(86) International application number: PCT/JP2023/040807
(87) International publication number: WO 2024/106394

(57) **Abstract**

A technique for selectively recognizing TNBC is provided. An antibody against a triple-negative breast cancer cell (TNBC) selectively recognizes a TNBC in comparison to a HER2-positive breast cancer cell and a hormone receptor-positive breast cancer cell.

## Description

### TECHNICAL FIELD

The present invention relates to an antibody, and particularly to a VHH antibody against a triple-negative breast cancer (TNBC) cell.

### BACKGROUND ART

VHH antibodies are heavy-chain antibodies contained in the sera of camelids (e.g., Bactrian camels, Arabian camels, llamas, alpacas, and the like). The VHH antibodies have been attracting attention because they have a high ability to recognize antigens as well as high stability while being capable of specifically binding to the antigens similarly to common antibodies (see, for example, Patent Literature 1 to Patent Literature 3). The VHH antibodies are generally composed of a short amino-acid chain, and are thus also excellent in that they can be easily produced. Patent Literature 4 discloses a method for logically identifying the amino acid sequence of a VHH antibody that recognizes a target substance to produce the VHH antibody. Furthermore, as described in Patent Literature 1 and Patent Literature 3, modification of a VHH antibody (e.g., introduction of mutation into the framework regions located upstream and downstream of the complementarity determining region (CDR) of a VHH antibody that recognizes an antigen) and introduction of the amino acid sequence of the CDR into another type of antibody are also conducted.

### CITATION LIST

### PATENT LITERATURE

PTL1: Japanese Patent Laid-Open No. 2005-520494
PTL2: Japanese Patent Laid-Open No. 2017-14112
PTL3: Japanese Patent Laid-Open No. 2013-506411
PTL4: International Publication No. 2019/230823

### SUMMARY OF INVENTION

### TECHNICAL PROBLEM

There is a limitation on effective therapy for triple-negative breast cancer (TNBC) out of the breast cancer subtypes. Accordingly, there is a social demand for a method for diagnosing TNBC or a method for treating TNBC.

It is an object of the present invention to provide a technique for selectively recognizing TNBC.

### SOLUTION TO PROBLEM

According to an embodiment of the present invention, an antibody against a triple-negative breast cancer cell (TNBC) selectively recognizes a TNBC in comparison to a HER2-positive breast cancer cell and a hormone receptor-positive breast cancer cell.

### ADVANTAGEOUS EFFECTS OF INVENTION

It is possible to provide a technique for selectively recognizing TNBC.

### DESCRIPTION OF EMBODIMENTS

Hereinafter, embodiments will be described in detail. Note, the following embodiments are not intended to limit the scope of the claimed invention, and limitation is not made to an invention that requires a combination of all features described in the embodiments. Two or more of the multiple features described in the embodiments may be combined as appropriate. Furthermore, the same reference numerals are given to the same or similar configurations, and redundant description thereof is omitted.

An antibody according to an embodiment of the present invention is a VHH antibody having an amino acid sequence selected from the group consisting of SEQ ID NOs: 1 to 17.

An antibody according to another embodiment of the present invention is an anti-TNBC antibody having at least 90% identity with an amino acid sequence selected from the group consisting of SEQ ID NOs: 1 to 17. In an embodiment, the identity therebetween is 95% or more, 98% or more, or 99% or more. The amino acid sequence identity is evaluated using a protein homology analysis program, NCBI blastp. In an embodiment, the antibody is a VHH antibody.

An antibody according to another embodiment of the present invention is an anti-TNBC antibody that has, as CDRs of the antibody:
CDR1 represented by SEQ ID NO: 18, CDR2 represented by SEQ ID NO: 19, and CDR3 represented by SEQ ID NO: 20;
CDR1 represented by SEQ ID NO: 21, CDR2 represented by SEQ ID NO: 22, and CDR3 represented by SEQ ID NO: 23;
CDR1 represented by SEQ ID NO: 24, CDR2 represented by SEQ ID NO: 25, and CDR3 represented by SEQ ID NO: 26;
CDR1 represented by SEQ ID NO: 27, CDR2 represented by SEQ ID NO: 28, and CDR represented by SEQ ID NO: 29;
CDR1 represented by SEQ ID NO: 30, CDR2 represented by SEQ ID NO: 31, and CDR3 represented by SEQ ID NO: 32;
CDR1 represented by SEQ ID NO: 33, CDR2 represented by SEQ ID NO: 34, and CDR represented by SEQ ID NO: 35;
CDR1 represented by SEQ ID NO: 36, CDR2 represented by SEQ ID NO: 37, and CDR3 represented by SEQ ID NO: 38;
CDR1 represented by SEQ ID NO: 39, CDR2 represented by SEQ ID NO: 40, and CDR represented by SEQ ID NO: 41;
CDR1 represented by SEQ ID NO: 42, CDR2 represented by SEQ ID NO: 43, and CDR3 represented by SEQ ID NO: 44;
CDR1 represented by SEQ ID NO: 45, CDR2 represented by SEQ ID NO: 46, and CDR3 represented by SEQ ID NO: 47;
CDR1 represented by SEQ ID NO: 48, CDR2 represented by SEQ ID NO: 49, and CDR3 represented by SEQ ID NO: 50;
CDR1 represented by SEQ ID NO: 51, CDR2 represented by SEQ ID NO: 52, and CDR3 represented by SEQ ID NO: 53;
CDR1 represented by SEQ ID NO: 54, CDR2 represented by SEQ ID NO: 55, and CDR3 represented by SEQ ID NO: 56;
CDR1 represented by SEQ ID NO: 57, CDR2 represented by SEQ ID NO: 58, and CDR3 represented by SEQ ID NO: 59;
CDR1 represented by SEQ ID NO: 60, CDR2 represented by SEQ ID NO: 61, and CDR3 represented by SEQ ID NO: 62;
CDR1 represented by SEQ ID NO: 63, CDR2 represented by SEQ ID NO: 64, and CDR3 represented by SEQ ID NO: 65; or
CDR1 represented by SEQ ID NO: 66, CDR2 represented by SEQ ID NO: 67, and CDR3 represented by SEQ ID NO: 68. In an embodiment, the antibody is a VHH antibody.

An antibody according to another embodiment of the present invention is an anti-TNBC antibody that has, as CDRs of the antibody:
a CDR1 having an amino acid sequence consisting of an amino acid sequence represented by SEQ ID NO: 18 with deletion, substitution, or addition of 0 or 1 amino acid, a CDR2 having an amino acid sequence consisting of an amino acid sequence represented by SEQ ID NO: 19 with deletion, substitution, or addition of 0 or 1 amino acid, and a CDR3 having an amino acid sequence consisting of an amino acid sequence represented by SEQ ID NO: 20 with deletion, substitution, or addition of 0 or 1 amino acid;
a CDR1 having an amino acid sequence consisting of an amino acid sequence represented by SEQ ID NO: 21 with deletion, substitution, or addition of 0 or 1 amino acid, a CDR2 having an amino acid sequence consisting of an amino acid sequence represented by SEQ ID NO: 22 with deletion, substitution, or addition of 0 or 1 amino acid, and a CDR3 having an amino acid sequence consisting of an amino acid sequence represented by SEQ ID NO: 23 with deletion, substitution, or addition of 0 or 1 amino acid;
a CDR1 having an amino acid sequence consisting of an amino acid sequence represented by SEQ ID NO: 24 with deletion, substitution, or addition of 0 or 1 amino acid, a CDR2 having an amino acid sequence consisting of an amino acid sequence represented by SEQ ID NO: 25 with deletion, substitution, or addition of 0 or 1 amino acid, and a CDR3 having an amino acid sequence consisting of an amino acid sequence represented by SEQ ID NO: 26 with deletion, substitution, or addition of 0 or 1 amino acid;
a CDR1 having an amino acid sequence consisting of an amino acid sequence represented by SEQ ID NO: 27 with deletion, substitution, or addition of 0 or 1 amino acid, a CDR2 having an amino acid sequence consisting of an amino acid sequence represented by SEQ ID NO: 28 with deletion, substitution, or addition of 0 or 1 amino acid, and a CDR3 having an amino acid sequence consisting of an amino acid sequence represented by SEQ ID NO: 29 with deletion, substitution, or addition of 0 or 1 amino acid;
a CDR1 having an amino acid sequence consisting of an amino acid sequence represented by SEQ ID NO: 30 with deletion, substitution, or addition of 0 or 1 amino acid, a CDR2 having an amino acid sequence consisting of an amino acid sequence represented by SEQ ID NO: 31 with deletion, substitution, or addition of 0 or 1 amino acid, and a CDR3 having an amino acid sequence consisting of an amino acid sequence represented by SEQ ID NO: 32 with deletion, substitution, or addition of 0 or 1 amino acid;
a CDR1 having an amino acid sequence consisting of an amino acid sequence represented by SEQ ID NO: 33 with deletion, substitution, or addition of 0 or 1 amino acid, a CDR2 having an amino acid sequence consisting of an amino acid sequence represented by SEQ ID NO: 34 with deletion, substitution, or addition of 0 or 1 amino acid, and a CDR3 having an amino acid sequence consisting of an amino acid sequence represented by SEQ ID NO: 35 with deletion, substitution, or addition of 0 or 1 amino acid;
a CDR1 having an amino acid sequence consisting of an amino acid sequence represented by SEQ ID NO: 36 with deletion, substitution, or addition of 0 or 1 amino acid, a CDR2 having an amino acid sequence consisting of an amino acid sequence represented by SEQ ID NO: 37 with deletion, substitution, or addition of 0 or 1 amino acid, and a CDR3 having an amino acid sequence consisting of an amino acid sequence represented by SEQ ID NO: 38 with deletion, substitution, or addition of 0 or 1 amino acid;
a CDR1 having an amino acid sequence consisting of an amino acid sequence represented by SEQ ID NO: 39 with deletion, substitution, or addition of 0 or 1 amino acid, a CDR2 having an amino acid sequence consisting of an amino acid sequence represented by SEQ ID NO: 40 with deletion, substitution, or addition of 0 or 1 amino acid, and a CDR3 having an amino acid sequence consisting of an amino acid sequence represented by SEQ ID NO: 41 with deletion, substitution, or addition of 0 or 1 amino acid;
a CDR1 having an amino acid sequence consisting of an amino acid sequence represented by SEQ ID NO: 42 with deletion, substitution, or addition of 0 or 1 amino acid, a CDR2 having an amino acid sequence consisting of an amino acid sequence represented by SEQ ID NO: 43 with deletion, substitution, or addition of 0 or 1 amino acid, and a CDR3 having an amino acid sequence consisting of an amino acid sequence represented by SEQ ID NO: 44 with deletion, substitution, or addition of 0 or 1 amino acid;
a CDR1 having an amino acid sequence consisting of an amino acid sequence represented by SEQ ID NO: 45 with deletion, substitution, or addition of 0 or 1 amino acid, a CDR2 having an amino acid sequence consisting of an amino acid sequence represented by SEQ ID NO: 46 with deletion, substitution, or addition of 0 or 1 amino acid, and a CDR3 having an amino acid sequence consisting of an amino acid sequence represented by SEQ ID NO: 47 with deletion, substitution, or addition of 0 or 1 amino acid;
a CDR1 having an amino acid sequence consisting of an amino acid sequence represented by SEQ ID NO: 48 with deletion, substitution, or addition of 0 or 1 amino acid, a CDR2 having an amino acid sequence consisting of an amino acid sequence represented by SEQ ID NO: 49 with deletion, substitution, or addition of 0 or 1 amino acid, and a CDR3 having an amino acid sequence consisting of an amino acid sequence represented by SEQ ID NO: 50 with deletion, substitution, or addition of 0 or 1 amino acid;
a CDR1 having an amino acid sequence consisting of an amino acid sequence represented by SEQ ID NO: 51 with deletion, substitution, or addition of 0 or 1 amino acid, a CDR2 having an amino acid sequence consisting of an amino acid sequence represented by SEQ ID NO: 52 with deletion, substitution, or addition of 0 or 1 amino acid, and a CDR3 having an amino acid sequence consisting of an amino acid sequence represented by SEQ ID NO: 53 with deletion, substitution, or addition of 0 or 1 amino acid;
a CDR1 having an amino acid sequence consisting of an amino acid sequence represented by SEQ ID NO: 54 with deletion, substitution, or addition of 0 or 1 amino acid, a CDR2 having an amino acid sequence consisting of an amino acid sequence represented by SEQ ID NO: 55 with deletion, substitution, or addition of 0 or 1 amino acid, and a CDR3 having an amino acid sequence consisting of an amino acid sequence represented by SEQ ID NO: 56 with deletion, substitution, or addition of 0 or 1 amino acid;
a CDR1 having an amino acid sequence consisting of an amino acid sequence represented by SEQ ID NO: 57 with deletion, substitution, or addition of 0 or 1 amino acid, a CDR2 having an amino acid sequence consisting of an amino acid sequence represented by SEQ ID NO: 58 with deletion, substitution, or addition of 0 or 1 amino acid, and a CDR3 having an amino acid sequence consisting of an amino acid sequence represented by SEQ ID NO: 59 with deletion, substitution, or addition of 0 or 1 amino acid;
a CDR1 having an amino acid sequence consisting of an amino acid sequence represented by SEQ ID NO: 60 with deletion, substitution, or addition of 0 or 1 amino acid, a CDR2 having an amino acid sequence consisting of an amino acid sequence represented by SEQ ID NO: 61 with deletion, substitution, or addition of 0 or 1 amino acid, and a CDR3 having an amino acid sequence consisting of an amino acid sequence represented by SEQ ID NO: 62 with deletion, substitution, or addition of 0 or 1 amino acid;
a CDR1 having an amino acid sequence consisting of an amino acid sequence represented by SEQ ID NO: 63 with deletion, substitution, or addition of 0 or 1 amino acid, a CDR2 having an amino acid sequence consisting of an amino acid sequence represented by SEQ ID NO: 64 with deletion, substitution, or addition of 0 or 1 amino acid, and a CDR3 having an amino acid sequence consisting of an amino acid sequence represented by SEQ ID NO: 65 with deletion, substitution, or addition of 0 or 1 amino acid; or
a CDR1 having an amino acid sequence consisting of an amino acid sequence represented by SEQ ID NO: 66 with deletion, substitution, or addition of 0 or 1 amino acid, a CDR2 having an amino acid sequence consisting of an amino acid sequence represented by SEQ ID NO: 67 with deletion, substitution, or addition of 0 or 1 amino acid, a CDR3 having an amino acid sequence consisting of an amino acid sequence rep and resented by SEQ ID NO: 68 with deletion, substitution, or addition of 0 or 1 amino acid. In an embodiment, the antibody is a VHH antibody.

An antibody according to an embodiment of the present invention is an antibody that specifically binds to a triple-negative breast cancer cell (TNBC). The antibody according to the embodiment is an antibody against a triple-negative breast cancer cell (TNBC) that selectively recognizes a TNBC in comparison to a HER2-positive breast cancer cell and a hormone receptor-positive breast cancer cell. The wording "selectively recognize a TNBC" means that the TNBC recognized in distinction from the HER2-positive breast cancer cell and the hormone receptor-positive breast cancer cell through cell staining using the antibody. The antibody according to the embodiment of the present invention can bind to an antigen that is expressed in a triple-negative breast cancer cell (TNBC) specifically or in a larger amount.

In this specification, the HER2-positive breast cancer cell is a breast cancer cell in which HER2 is not overexpressed. The hormone receptor-positive breast cancer cell is a breast cancer cell in which the estrogen receptor (ER) and the progesterone receptor (PR) are not detected. The TNBC is a breast cancer cell in which the estrogen receptor (ER) and the progesterone receptor (PR) are not detected and HER2 is not overexpressed. Breast cancer cells can be classified into the TNBC, the HER2-positive breast cancer cell, and the hormone receptor-positive breast cancer cell in accordance with a common diagnosis method.

The antibody according to the embodiment does not substantially bind to the HER2-positive breast cancer cell and the hormone receptor-positive breast cancer cell. The wording "the antibody does not substantially bind to the breast cancer cells" means that the breast cancer cells are not stained through cell staining using the antibody.

An antibody according to an embodiment is an anti-TNBC antibody that selectively recognizes a TNBC in comparison to a normal breast cell. The wording "selectively recognize a TNBC" means that the TNBC rather than the normal breast cell can be recognized through cell staining using the antibody. The normal breast cell is a cell in the breast parenchymal that has not cancerated, and specifically an epithelial cell that includes a mammary acinar cell and a mammary duct cell. The antibody according to the embodiment does not substantially bind to the normal breast cell.

In this specification, the meaning of the term "antibody" follows the common usage of this term. The antibody may be a polyclonal antibody or a monoclonal antibody. In the embodiment, the antibody is an isolated monoclonal antibody. The antibody includes a multimer and also includes an antigen-binding fragment. For example, the antibodies according to the embodiment may form a dimer via a linker. In a specific example, the VHH antibody according to the embodiment may form a homodimer together with a VHH antibody having the same sequence, or may form a heterodimer with a VHH antibody having a different sequence. Examples of the antigen-binding fragment include a minibody, a single-chain antibody (scFV), a variable region fragment (Fv or Fd), Fab, F(ab)₂, and the like.

In an embodiment, the antibody is a VHH antibody. The VHH antibody is a heavy-chain antibody that is also called a single-domain antibody or a nanobody. The VHH is generally constituted by regions FR1, CDR1, FR2, CDR2, FR3, CDR3, and FR4. FR1 to FR4 are each called a framework region. CDR1 to CDR3, which are each located between the framework regions, are hypervariable regions, and are each called a complementarity determining region. In general, it is considered that CDR1 to CDR3 recognize an antigen and specifically bind to the antigen. These regions can be identified in accordance with the descriptions in Elvin A. Kabat et al. "Sequence of proteins of immunological interest", NIH publication No. 91-3242, and L. Riechmann et al. "Single domain antibodies: comparison of camel VH and camelised human VH domains", J. Immunol. Methods, 1999, 231, 25-38.

Such VHH antibodies are produced by camelids such as an alpaca. Meanwhile, a VHH antibody having a certain amino acid sequence can be easily produced using a method of, for example, synthesizing a gene and introducing the gene as a plasmid into E. coli.

Meanwhile, the antibody including the VHH antibody may be a human antibody, a humanized antibody, or a chimeric antibody. For example, the VHH antibody may be humanized. Specifically, in the framework regions of the VHH antibody, one or more amino acid residues may be deleted, substituted or added according to the amino acid sequence of the VH domain of the human antibody. The VHH antibody may be optimized using another method. In the embodiment, the antibody may be a bispecific antibody. That is to say, the antibody may specifically bind to another antigen in addition to the TNBC. An example of the bispecific antibody is an antibody that specifically binds to the TNBC and CD3.

An additional molecule may be linked to the antibody according to the embodiment. The molecule that may be linked to the antibody may be a polypeptide chain (e.g., a VHH antibody, an immunoglobulin, or a fragment thereof, or an enzyme), a drug (e.g., an antiviral agent, an antitumor agent, or a cytotoxic drug), a luminescent substance, another chemical substance (e.g., polyethylene glycol), or the like. The cytotoxic drug refers to a drug that can injure cells or suppress proliferation of cells. The antitumor agent refers to a drug that can selectively injure tumor cells or suppress proliferation of tumor cells. These molecules may be linked to the VHH antibody via a linker sequence. In this specification, the antibody also includes an antibody modified using a method for linking a molecule in such a manner, or the like.

The antibody that recognizes a TNBC according to the embodiment can be identified by immunizing an animal against the TNBC and panning, using the TNBC, antibodies induced by the immunization. Identifying the amino acid sequence of the thus-identified antibody makes it possible to produce a monoclonal antibody that recognizes the TNBC. When a VHH antibody is identified, camelids such as an alpaca or cartilaginous fish such as a shark can be used as the animal. The production of an antibody having a certain amino acid sequence can be achieved using a method for transfecting a plasmid encoding the antibody into E. coli or a CHO cell, or the like.

The amino acid sequence of the antibody according to the embodiment can be identified in accordance with the method described in International Publication No. 2019/230823. For example, the amino acid sequence of an antibody that specifically recognizes the TNBC can be identified by using the TNBC as a target substance and a breast cancer cell other than the TNBC as a control substance, and subtracting the amino acid sequence of an antibody that recognizes the subject substance from the amino acid sequence of an antibody that recognizes the TNBC. Specifically, an animal to be used to produce an antibody is immunized with the TNBCs, and the gene sequences of lymphocytes that produce a group of antibodies induced in the animal are sequenced. Then, phages into which the genes having the sequenced gene sequences have been introduced are allowed to interact with the TNBCs, and the amino acid sequence corresponding to the gene sequence introduced into the phage that has bound to the TNBC is identified as the amino acid sequence of an antibody that recognizes the TNBC. Similarly, phages into which the genes having the sequenced gene sequences have been introduced are allowed to interact with the control substance, and the amino acid sequence corresponding to the gene sequence introduced into the phage that has bound to the control substance is identified as the amino acid sequence of an antibody that recognizes the control substance. Here, a next-generation sequencer can be used to sequence the gene sequences.

Then, antibodies that selectively recognize the TNBC in comparison to the control substance are identified by comparing the amino acid sequences of the antibody group that recognizes the TNBC and the amino acid sequences of the antibody group that recognizes the control substance. In order to facilitate the comparison of the amino acid sequences and discovery of an antibody that selectively recognizes the TNBC, the antibodies having similar amino acid sequences may be grouped through clustering conducted under predetermined conditions, followed by the comparison of the amino acid sequences between the groups.

In the comparison of the amino acid sequences, it is possible to compare the occurrence frequency of a certain amino acid sequence (or group) in the antibody group that recognizes the TNBC and the occurrence frequency of the certain amino acid sequence (or group) in the antibody group that recognizes the control substance. An amino acid sequence (or group) that occurs relatively more frequently in the antibody group that recognizes the TNBC than in the antibody group that recognizes the control substance can be preferentially identified as an antibody that recognizes the TNBC. For example, antibodies that are included in the antibody group that recognizes the TNBC and the number of which in the antibody group that recognizes the control substance is smaller than or equal to a predetermined criterion can be identified as antibodies that selectively recognize the TNBC. In an example, a group C₀ is a group of antibodies that are included in the antibody group that recognizes the TNBC (this group is referred to as a "group A") and that are not included in the antibody group that recognizes the control substance (this group is referred to as a "group B"). A group C₁₀ is a group of antibodies that are included in the group A and the number of which in the group B is one tenth or smaller of that in the group A. A group C_{1/2} is a group of antibodies that are included in the group A and the number of which in the group B is half or smaller of that in the group A. Antibodies included in group C₀, group C₁₀, and group C_{1/2} can be selected as antibodies that selectively recognize the TNBC.

The antibody as described above that specifically binds to the TNBC can be used for diagnosis of the TNBC or treatment of the TNBC. For example, an antibody that selectively recognizes the TNBC in comparison to the HER2-positive breast cancer cell and the hormone receptor-positive breast cancer cell can be used to diagnose the breast cancer subtype. For example, an antibody labeled with an enzyme or luminescent substance can be used for such diagnosis. An antibody that specifically binds to the TNBC can be used to exhibit an antibody-dependent cell toxicity to the TNBC, or can be used to produce an antibody-drug complex for attacking the TNBC.

### Examples

Hereinafter, the present invention will be described in detail by use of examples, but the present invention is not limited to these examples. Antibodies were identified as follows in accordance with the method described in International Publication No. 2019/230823.

### Example 1: Induction of Immune Response in Alpaca

An alpaca was immunized with seven types of breast cancer cell lines (SKBR3, MDA-MB-231, HS578, BT549, MDA-MB-436, MDA-MB-468, and HCC1937). The alpaca was immunized in accordance with the common method.

### Example 2: Construction of VHH Antibody Phage Library

Lymphocytes were collected from the blood of one alpaca in which the immune response was induced in accordance with Example 1. With reference to known methods, a group of VHH antibody genes (VHH antibody gene library) included in the lymphocytes was obtained and a group of phages (VHH antibody phage library) having the group of gene sequences was constructed.

Specifically, a blood sample was collected from the jugular vein, and peripheral blood mononuclear cells (PBMCs) were obtained through sucrose density gradient using Ficoll (manufactured by Nacalai Tesque, Inc.). After washed with PBS, the PBMCs were suspended in the RNAlater solution (manufactured by Thermo Fisher Scientific). Then, total RNA was separated from the PBMC sample (Direct-Zol RNA MiniPrep, manufactured by Zymo Research Corporation). Complementary DNA was synthesized using 1 µg of the total RNA as a template, a random hexamer primer, and SuperScript II Reverse Transcriptase (manufactured by Thermo Fisher Scientific). The heavy-chain variable domain-coding region was amplified using LA taq Polymerase (manufactured by Takara Bio Inc.) and two primers (CALL001: 5'-GTCCTGGCTGCTCTTCTACAAGG-3' (SEQ ID NO: 69) and CALL002: 5'-GGTACGTGCTGTTGAACTGTTCC-3' (SEQ ID NO: 70)) purified through PAGE. Fragments of the amplified heavy-chain variable domain-coding gene were separated using the 1.5% low-melting-point agarose gel (manufactured by Lonza Group AG), and a band with a lower molecular weight of about 700 base pairs corresponding to an immunoglobulin having only a heavy chain was extracted (QIAquick Gel Extraction Kit, manufactured by QIAGEN N.V). Nested PCR was conducted using VHH-PstI-For and VHH-BstEII-Rev primers in order to amplify the VHH domain-coding gene, and the amplified gene was subcloned into a pMES4 phagemid vector (GeneArt DNA Synthesis, manufactured by Thermo Fisher Scientific). Electroporation-competent E. coli TG1 cells (manufactured by Agilent Technologies Japan, Ltd.) were transformed using the ligated plasmid at a low temperature, and the titer was checked by conducting the limiting dilution method such that the colony-forming unit was maintained to be higher than 10⁷/µL. Colonies were collected from 8-mL cultured cells and pooled, and were then stored in a frozen glycerol stock as a mother library.

### Example 3: Selection of VHH Antibody Interacting with Target Substance, and Example 4: Selection of VHH Antibody Interacting with Control Substance

Eight types of TNBC cell lines (MDA-MB-231, HS578, BT549, MDA-MB-436, MDA-MB-468, HCC1937, HCC1599, and HCC38) were used as target substances. Phages capable of binding to these cell lines were enriched by allowing the group of phages obtained in Example 2 to interact with the cell lines serving as the target substances.

E. coli for phage infection (TG1 competent cells) was infected with the collected phages, and was then stored as an E. coli library. The group of gene sequences of the VHH antibodies that bound to magnetic beads to which the target substances had been linked were present in the form of plasmids in the constructed E. coli library, and the plasmids were purified using a method recommended by the manufacturer (QIAGEN N.V).

The HER2-positive breast cancer cell line (SKBR3) and the hormone receptor-positive breast cancer cell line (T47D) were used as control substances. In the same manner as in Example 3, phages capable of binding to these cell lines were enriched by allowing the group of phages obtained in Example 2 to interact with the cell lines serving as the subject substances. Also, the collected phages were stored as an E. coli library. Furthermore, plasmids that included the VHH antibody gene sequences were purified.

Specifically, each of the eluted phage-containing solutions was neutralized using a solution of a protein inhibitor cocktail (cOmplete, EDTA-free, Protease inhibitor cocktail tablets, manufactured by Roche Diagnostics K.K.) diluted with PBS, and the diluted solution was used to cause infection to the TG1 electroporation-competent cells. The infected cells were cultured in the LB broth (manufactured by Miller) containing ampicillin (manufactured by Nacalai Tesque, Inc.) at a concentration of 100 µg/mL at 37°C overnight for screening. The screened phagemids were collected using the QIAprep mini-prep kit (manufactured by QIAGEN N.V.).

### Example 5: Sequencing of Amino Acid Sequence of VHH Antibody

The groups of VHH antibody gene sequences obtained in Examples 3 and 4 were sequenced through next-generation sequence analysis. Specifically, a portion of 500 or less base pairs in the VHH antibody gene sequence was selected as a target region, and the gene sequences in this region were sequenced. 5'gaaatacctattgcctacggc (5' side, SEQ ID NO: 71) and 5'ggaaccgtagtccggaacgtc (3' side, SEQ ID NO: 72) were designed as primers for amplification of the target region. The target region, which was a portion of 500 or less base pairs in the VHH antibody gene sequence, was amplified using this primer set in polymerase chain reaction (PCR). Furthermore, the gene sequence of the target region was sequenced by conducting the illumina_MiSeq_Amplicon_Deep_Sequence method in the manner recommended by the manufacturer (Illumina Inc.) using a primer set that included primers obtained by fusing, to the primers above, 5'TCGTCGGCAGCGTCAGATGTGTATAAGAGACAG- (SEQ ID NO: 73) and 5'GTCTCGTGGGCTCGGAGATGTGTATAAGAGACAG- (SEQ ID NO: 74), which are index sequences specific to the next-generation sequencer.

The thus-sequenced group of gene sequences was translated to a group of amino acid sequences using commercially available analysis software (Genetyx). In the group of gene sequences and the group of amino acid sequences, selected were gene sequences and amino acid sequences in which the terminal sequence was completely the same as the sequences of the primers used, in which the target region had a length of 401 or more base pairs, with two or more read counts, and including a His-tag sequence (6×His) included in the vector sequence. Furthermore, those having different gene sequences but the same amino acid sequence were also added, and the amino acid sequences were listed in decreasing order of the occurrence frequency (read counts).

Specifically, the mother library and the VHH-coding regions in sub-libraries after one round of the target enrichment were amplified through PCR, and the amplified products were purified using the AMPure XP beads (Beckman Coulter, Inc.). Then, a dual index library was prepared and sequenced with Illumina MiSeq (manufactured by Illumina Inc.) using MiSeq Reagent Kit v3 under the conditions of "paired" and "300 bp read". Thus, about 100,000 paired reads were generated for each sub-library. The adaptor sequences were trimmed from the raw data of the reads using cutadapt v1.18 (Martin, M. Cutadapt removes adapter sequences from high-throughput sequencing reads. EMBnet.journal, 17, 10-12, doi: 10.14806/ej.17.1.200 (2011).), and then low-quality reads were removed using Trimmatic v0.39 (Bolger, A. M., Lohse, M. & Usadel, B. Trimmomatic: a flexible trimmer for Illumina sequence data. Bioinformatics 30, 2114-2120, doi: 10.1093/bioinformatics/btu170 (2014)). The remaining paired reads were merged using fastq-join (Aronesty, E. Comparison of Sequencing Utility Programs. The Open Bioinformatics Journal 7, 1-8, doi: 10.2174/1875036201307010001 (2013)) and translated into amino acid sequences using EMBOSS v6.6.0.0 (Rice, P., Longden, I. & Bleasby, A. EMBOSS: the European Molecular Biology Open Software Suite. Trends in genetics: TIG 16, 276-277, doi: 10.1016/s0168-9525(00)02024-2 (2000)).

### Example 6: Subtraction of Amino Acid Sequences of VHH Antibodies Interacting with Control Substance from Amino Acid Sequences of VHH Antibodies Interacting with Target Substance

Unique sequences were counted in each library using a custom python script that is a combination of seqkit v0.10.1 (Shen, W., Le, S., Li, Y. & Hu, F. SeqKit: A Cross-Platform and Ultrafast Toolkit for FASTA/Q File Manipulation. PLOS ONE 11, e0163962, doi: 10.1371/journal.pone.0163962 (2016)) and usearch v.11 (Edgar, R. C. Search and clustering orders of magnitude faster than BLAST. Bioinformatics 26, 2460-2461, doi: 10.1093/bioinformatics/btq461 (2010)). The enrichment score of each clone was analyzed by conducting a chi-square test to calculate a P value between the abundance ratios in the bio-panned sub-libraries. As a result, ten clones were selected and the enrichment score for each of the ten clones was higher in the sub-library panned using the TNBC cell line than in the sub-libraries panned using the HER2-positive breast cancer cell and the hormone receptor-positive breast cancer cell line. Thus, ten clones of VHH antibodies (Clone Nos. 89, 777, 500, 72, 172, 63, 286, 673, 1360, and 610) were identified.

The amino acid sequences of the clones are shown in Sequence Listing and are as follows. The amino acid sequences of CDRs of each clone are shown in parentheses as "(CDR1, CD2, CDR3)".
Clone No. 89: SEQ ID NO: 3 (SEQ ID NO: 24, SEQ ID NO: 25, SEQ ID NO: 26)
Clone No. 500: SEQ ID NO: 6 (SEQ ID NO: 33, SEQ ID NO: 34, SEQ ID NO: 35)
Clone No. 72: SEQ ID NO: 9 (SEQ ID NO: 42, SEQ ID NO: 43, SEQ ID NO: 44)
Clone No. 172: SEQ ID NO: 10 (SEQ ID NO: 45, SEQ ID NO: 46, SEQ ID NO: 47)
Clone No. 63: SEQ ID NO: 2 (SEQ ID NO: 21, SEQ ID NO: 22, SEQ ID NO: 23)
Clone No. 286: SEQ ID NO: 5 (SEQ ID NO: 30, SEQ ID NO: 31, SEQ ID NO: 32)
Clone No. 673: SEQ ID NO: 14 (SEQ ID NO: 57, SEQ ID NO: 58, SEQ ID NO: 59)
Clone No. 1360: SEQ ID NO: 7 (SEQ ID NO: 36, SEQ ID NO: 37, SEQ ID NO: 38)
Clone No. 610: SEQ ID NO: 13 (SEQ ID NO: 54, SEQ ID NO: 55, SEQ ID NO: 56)
Clone No. 777: SEQ ID NO: 4 (SEQ ID NO: 27, SEQ ID NO: 28, SEQ ID NO: 29)

Also, further seven clone VHH antibodies were identified using the same technique. The amino acid sequences of the clones are shown in Sequence Listing and are as follows. The amino acid sequences of CDRs of each clone are shown in parentheses as "(CDR1, CD2, CDR3)".
Clone No. 21: SEQ ID NO: 1 (SEQ ID NO: 18, SEQ ID NO: 19, SEQ ID NO: 20)
Clone No. 68: SEQ ID NO: 8 (SEQ ID NO: 39, SEQ ID NO: 40, SEQ ID NO: 41)
Clone No. 242: SEQ ID NO: 11 (SEQ ID NO: 48, SEQ ID NO: 49, SEQ ID NO: 50)
Clone No. 259: SEQ ID NO: 12 (SEQ ID NO: 51, SEQ ID NO: 52, SEQ ID NO: 53)
Clone No. 769: SEQ ID NO: 15 (SEQ ID NO: 60, SEQ ID NO: 61, SEQ ID NO: 62)
Clone No. 3511: SEQ ID NO: 16 (SEQ ID NO: 63, SEQ ID NO: 64, SEQ ID NO: 65)
Clone No. 13289: SEQ ID NO: 17 (SEQ ID NO: 66, SEQ ID NO: 67, SEQ ID NO: 68)

### Example 7: Synthesis of Artificial Genes Having Gene Sequences Encoding Identified VHH Antibodies

The amino acid sequences of the identified VHH antibodies were all composed of 200 or less amino acid residues, and the gene sequences encoding these amino acid sequences could be artificially synthesized as optimized gene sequences provided by the manufacturer (Eurofins Scientific) (the term "optimized gene sequences" as used herein refer to, for example, gene sequences that are easy to synthesis, that are less likely to be decomposed, that does not exhibit a base imbalance, that includes less repeated sequences, or that are less likely to form a secondary structure). In this example, genes encoding VHH antibody tandem dimers were synthesized. Methods for expressing a VHH antibody as a dimer or multimer and using it are well known as described in Japanese Patent Laid-Open No. 2010-534465 and the like. The artificially synthesized genes could be incorporated into any expression vector.

Specifically, the amino acid sequences of the identified VHH antibodies were linked via a (GGGGS)₄ linker (SEQ ID NO: 75) to produce tandem homodimers. Then, codon-optimized genes encoding these homodimers were synthesized.

### Example 8: Expression of VHH Antibody Proteins Encoded by Artificial Genes

Introducing, into E. coli (BL21 strain), the plasmids obtained by incorporating the artificial genes into expression vectors enabled expression of the VHH antibody proteins encoded by the artificial genes in E. coli. Specifically, the expression of the amino acid sequences encoded by the artificial genes was induced by adding IPTG to E. coli in a logarithmic growth phase. The expressed amino acid sequences (the VHH antibodies with a His tag) were purified through a nickel-immobilized resin column using a method recommended by the manufacturer (GE HealthCare).

### Example 9: Confirmation of Ability of VHH Antibodies to Recognize Antigen through Cell Staining

The VHH antibodies that had been identified in Example 6 and obtained in Examples 7 and 8 were fluorescently labeled, and cells were stained with these VHH antibodies. It was confirmed that each of the ten clone VHH antibodies stained at least one type of TNBC cell.

Specifically, the VHH antibody of Clone No. 89 stained MDA-MB-231, BT549, MDA-MB-436, HCC1937, HCC1599, and HCC38 out of eight types of TNBC cell lines (MDA-MB-231, HS578, BT549, MDA-MB-436, MDA-MB-468, HCC1937, HCC1599, and HCC38). Also, the VHH antibody of Clone No. 777 stained MDA-MB-231, BT549, MDA-MB-436, HCC1937, HCC1599, and HCC38 out of eight types of TNBC cell lines above. Meanwhile, both the VHH antibody of Clone No. 89 and the VHH antibody of Clone No. 777 did not stain the HER2-positive breast cancer cell lines (SKBR3 and BT474), the hormone receptor-positive breast cancer cell line (MCF7), and cancer cell lines (HEK, HeLa, and A431) other than breast cancer cell lines.

The VHH antibodies of Clone Nos. 500, 72, 172, 63, 286, 673, 1360, and 610 stained the TNBC cell lines as follows.
Clone No. 500: MDA-MB-231, BT549, and HCC1599
Clone No. 72: MDA-MB-231 and HCC1599
Clone No. 172: MDA-MB-231
Clone No. 63: HS578, BT549, HCC1937, and HCC1599
Clone No. 286: MDA-MB-231, HS578, BT549, MDA-MB-436, HCC1599, and HCC38
Clone No. 673: MDA-MB-231, BT549, MDA-MB-436, HCC1937, and HCC1599
Clone No. 1360: MDA-MB-231, MDA-MB-436, HCC1599, and HCC38
Clone No. 610: MDA-MB-436

It was confirmed that the further seven clone VHH antibodies (21, 68, 242, 259, 769, 3511, and 13289) stained at least one type of TNBC cell.

Note that staining of a cancer cell line (HEK293T) other than breast cancer cell lines using the 17 types of clones above all yielded negative results.

It was confirmed that, when used to stain human breast cancer frozen tissue arrays (BioChain Institute Inc.; Catalog No.: T6235086-5), the VHH antibody of Clone No. 89 and the VHH antibody of Clone No. 777 could stain breast cancer tissues of six cases and four cases, respectively.

Furthermore, when used to stain human normal frozen tissue arrays (BioChain Institute Inc.; Catalog No.: T6234701-2), the VHH antibody of Clone No. 89 and the VHH antibody of Clone No. 777 did not broadly stain the tissues of the breast.

As described above, it was confirmed that, with the methods above, antibodies that specifically bind to the TNBC, particularly the antibodies that selectively recognize the TNBC in comparison to the HER2-positive breast cancer cell and the hormone receptor-positive breast cancer cell, and antibodies that selectively recognize the TNBC in comparison to a normal breast cell can be obtained. It can also be understood that repeating the methods above makes it possible to obtain antibodies that specifically bind to various TNBCs within a rational experimental scope.

The invention is not limited to the foregoing embodiments, and various variations/changes are possible within the spirit of the invention.

## Claims

1. An antibody against a triple-negative breast cancer cell (TNBC),
wherein the antibody selectively recognizes a TNBC in comparison to a HER2-positive breast cancer cell and a hormone receptor-positive breast cancer cell.

2. The antibody according to claim 1,
wherein the antibody does not substantially bind to a HER2-positive breast cancer cell and a hormone receptor-positive breast cancer cell.

3. An antibody against a triple-negative breast cancer cell (TNBC),
wherein the antibody selectively recognizes a TNBC in comparison to a normal breast cell.

4. The antibody according to claim 3,
wherein the antibody does not substantially bind to a normal breast cell.

5. The antibody according to any one of claims 1 to 4,
wherein the antibody is a VHH antibody.

6. A method for producing a monoclonal antibody that recognizes a triple-negative breast cancer cell (TNBC), the method comprising:
a step of immunizing an animal with a TNBC; and
a step of panning an antibody induced through immunization, using the TNBC.

7. A VHH antibody having an amino acid sequence selected from the group consisting of SEQ ID NOs: 1 to 17.

8. An anti-TNBC antibody, comprising:
(1) a CDR1 having an amino acid sequence consisting of an amino acid sequence represented by SEQ ID NO: 18 with deletion, substitution, or addition of 0 or 1 amino acid, a CDR2 having an amino acid sequence consisting of an amino acid sequence represented by SEQ ID NO: 19 with deletion, substitution, or addition of 0 or 1 amino acid, and a CDR3 having an amino acid sequence consisting of an amino acid sequence represented by SEQ ID NO: 20 with deletion, substitution, or addition of 0 or 1 amino acid;
(2) a CDR1 having an amino acid sequence consisting of an amino acid sequence represented by SEQ ID NO: 21 with deletion, substitution, or addition of 0 or 1 amino acid, a CDR2 having an amino acid sequence consisting of an amino acid sequence represented by SEQ ID NO: 22 with deletion, substitution, or addition of 0 or 1 amino acid, and a CDR3 having an amino acid sequence consisting of an amino acid sequence represented by SEQ ID NO: 23 with deletion, substitution, or addition of 0 or 1 amino acid;
(3) a CDR1 having an amino acid sequence consisting of an amino acid sequence represented by SEQ ID NO: 24 with deletion, substitution, or addition of 0 or 1 amino acid, a CDR2 having an amino acid sequence consisting of an amino acid sequence represented by SEQ ID NO: 25 with deletion, substitution, or addition of 0 or 1 amino acid, and a CDR3 having an amino acid sequence consisting of an amino acid sequence represented by SEQ ID NO: 26 with deletion, substitution, or addition of 0 or 1 amino acid;
(4) a CDR1 having an amino acid sequence consisting of an amino acid sequence represented by SEQ ID NO: 27 with deletion, substitution, or addition of 0 or 1 amino acid, a CDR2 having an amino acid sequence consisting of an amino acid sequence represented by SEQ ID NO: 28 with deletion, substitution, or addition of 0 or 1 amino acid, and a CDR3 having an amino acid sequence consisting of an amino acid sequence represented by SEQ ID NO: 29 with deletion, substitution, or addition of 0 or 1 amino acid;
(5) a CDR1 having an amino acid sequence consisting of an amino acid sequence represented by SEQ ID NO: 30 with deletion, substitution, or addition of 0 or 1 amino acid, a CDR2 having an amino acid sequence consisting of an amino acid sequence represented by SEQ ID NO: 31 with deletion, substitution, or addition of 0 or 1 amino acid, and a CDR3 having an amino acid sequence consisting of an amino acid sequence represented by SEQ ID NO: 32 with deletion, substitution, or addition of 0 or 1 amino acid;
(6) a CDR1 having an amino acid sequence consisting of an amino acid sequence represented by SEQ ID NO: 33 with deletion, substitution, or addition of 0 or 1 amino acid, a CDR2 having an amino acid sequence consisting of an amino acid sequence represented by SEQ ID NO: 34 with deletion, substitution, or addition of 0 or 1 amino acid, and a CDR3 having an amino acid sequence consisting of an amino acid sequence represented by SEQ ID NO: 35 with deletion, substitution, or addition of 0 or 1 amino acid;
(7) a CDR1 having an amino acid sequence consisting of an amino acid sequence represented by SEQ ID NO: 36 with deletion, substitution, or addition of 0 or 1 amino acid, a CDR2 having an amino acid sequence consisting of an amino acid sequence represented by SEQ ID NO: 37 with deletion, substitution, or addition of 0 or 1 amino acid, and a CDR3 having an amino acid sequence consisting of an amino acid sequence represented by SEQ ID NO: 38 with deletion, substitution, or addition of 0 or 1 amino acid;
(8) a CDR1 having an amino acid sequence consisting of an amino acid sequence represented by SEQ ID NO: 39 with deletion, substitution, or addition of 0 or 1 amino acid, a CDR2 having an amino acid sequence consisting of an amino acid sequence represented by SEQ ID NO: 40 with deletion, substitution, or addition of 0 or 1 amino acid, and a CDR3 having an amino acid sequence consisting of an amino acid sequence represented by SEQ ID NO: 41 with deletion, substitution, or addition of 0 or 1 amino acid;
(9) a CDR1 having an amino acid sequence consisting of an amino acid sequence represented by SEQ ID NO: 42 with deletion, substitution, or addition of 0 or 1 amino acid, a CDR2 having an amino acid sequence consisting of an amino acid sequence represented by SEQ ID NO: 43 with deletion, substitution, or addition of 0 or 1 amino acid, and a CDR3 having an amino acid sequence consisting of an amino acid sequence represented by SEQ ID NO: 44 with deletion, substitution, or addition of 0 or 1 amino acid;
(10) a CDR1 having an amino acid sequence consisting of an amino acid sequence represented by SEQ ID NO: 45 with deletion, substitution, or addition of 0 or 1 amino acid, a CDR2 having an amino acid sequence consisting of an amino acid sequence represented by SEQ ID NO: 46 with deletion, substitution, or addition of 0 or 1 amino acid, and a CDR3 having an amino acid sequence consisting of an amino acid sequence represented by SEQ ID NO: 47 with deletion, substitution, or addition of 0 or 1 amino acid;
(11) a CDR1 having an amino acid sequence consisting of an amino acid sequence represented by SEQ ID NO: 48 with deletion, substitution, or addition of 0 or 1 amino acid, a CDR2 having an amino acid sequence consisting of an amino acid sequence represented by SEQ ID NO: 49 with deletion, substitution, or addition of 0 or 1 amino acid, and a CDR3 having an amino acid sequence consisting of an amino acid sequence represented by SEQ ID NO: 50 with deletion, substitution, or addition of 0 or 1 amino acid;
(12) a CDR1 having an amino acid sequence consisting of an amino acid sequence represented by SEQ ID NO: 51 with deletion, substitution, or addition of 0 or 1 amino acid, a CDR2 having an amino acid sequence consisting of an amino acid sequence represented by SEQ ID NO: 52 with deletion, substitution, or addition of 0 or 1 amino acid, and a CDR3 having an amino acid sequence consisting of an amino acid sequence represented by SEQ ID NO: 53 with deletion, substitution, or addition of 0 or 1 amino acid;
(13) a CDR1 having an amino acid sequence consisting of an amino acid sequence represented by SEQ ID NO: 54 with deletion, substitution, or addition of 0 or 1 amino acid, a CDR2 having an amino acid sequence consisting of an amino acid sequence represented by SEQ ID NO: 55 with deletion, substitution, or addition of 0 or 1 amino acid, and a CDR3 having an amino acid sequence consisting of an amino acid sequence represented by SEQ ID NO: 56 with deletion, substitution, or addition of 0 or 1 amino acid;
(14) a CDR1 having an amino acid sequence consisting of an amino acid sequence represented by SEQ ID NO: 57 with deletion, substitution, or addition of 0 or 1 amino acid, a CDR2 having an amino acid sequence consisting of an amino acid sequence represented by SEQ ID NO: 58 with deletion, substitution, or addition of 0 or 1 amino acid, and a CDR3 having an amino acid sequence consisting of an amino acid sequence represented by SEQ ID NO: 59 with deletion, substitution, or addition of 0 or 1 amino acid;
(15) a CDR1 having an amino acid sequence consisting of an amino acid sequence represented by SEQ ID NO: 60 with deletion, substitution, or addition of 0 or 1 amino acid, a CDR2 having an amino acid sequence consisting of an amino acid sequence represented by SEQ ID NO: 61 with deletion, substitution, or addition of 0 or 1 amino acid, and a CDR3 having an amino acid sequence consisting of an amino acid sequence represented by SEQ ID NO: 62 with deletion, substitution, or addition of 0 or 1 amino acid;
(16) a CDR1 having an amino acid sequence consisting of an amino acid sequence represented by SEQ ID NO: 63 with deletion, substitution, or addition of 0 or 1 amino acid, a CDR2 having an amino acid sequence consisting of an amino acid sequence represented by SEQ ID NO: 64 with deletion, substitution, or addition of 0 or 1 amino acid, and a CDR3 having an amino acid sequence consisting of an amino acid sequence represented by SEQ ID NO: 65 with deletion, substitution, or addition of 0 or 1 amino acid; or
(17) a CDR1 having an amino acid sequence consisting of an amino acid sequence represented by SEQ ID NO: 66 with deletion, substitution, or addition of 0 or 1 amino acid, a CDR2 having an amino acid sequence consisting of an amino acid sequence represented by SEQ ID NO: 67 with deletion, substitution, or addition of 0 or 1 amino acid, and a CDR3 having an amino acid sequence consisting of an amino acid sequence represented by SEQ ID NO: 68 with deletion, substitution, or addition of 0 or 1 amino acid, or
(18) at least 90% identity with an amino acid sequence selected from the group consisting of SEQ ID NOs: 1 to 17.
